# EUROPÄISCHE PATENTSCHRIFT

(11) **EP 1 103 542 B1**
(45) Veröffentlichungstag und Bekanntmachung des Hinweises auf die Patenterteilung: **28.04.2004**
(21) Anmeldenummer: 00115875.7
(22) Anmeldetag: 25.07.2000
(51) Int. Cl.: C07C 231/12, C07C 213/02, A61K 7/13

(54) **Verfahren zur Herstellung von 1,4-Diamino-2-methoxymethyl-benzol und dessen Salzen sowie die Verwendung dieser Verbindungen in Färbemitteln für Keratinfasern**
Process for the preparation of 1,4-diamino-2-methoxymethyl-benzene and its salts and the use of these compounds in dyeing compositions for keratinous fibres
Procédé pour la préparation de 1,4-diamino-2-méthoxyméthyl-benzène et ses sels et l' utilisation de ces composés dans une composition de teinture pour fibres kératiniques

(30) Priorität: 29.11.1999 DE 19957282
(43) Veröffentlichungstag der Anmeldung: 30.05.2001
(73) Patentinhaber: Wella Aktiengesellschaft, 64274 Darmstadt (DE)
(72) Erfinder: Göttel, Otto, Dr., 1723 Marly (CH); Pirrello, Aline, 1762 Givisiez (CH); Hayoz, André, 1724 Senèdes (CH); Braun, Hans-Jürgen, 3182 Überstorf (CH)

(56) Entgegenhaltungen:
- WO-A-99/11228
- DE-A- 2 741 762

## Beschreibung

Gegenstand der vorliegenden Anmeldung ist ein Verfahren zur Herstellung von 1,4-Diamino-2-methoxymethyl-benzol sowie dessen physiologisch verträglichen Salzen mit organischen oder anorganischen Säuren. Ein weiterer Gegenstand ist die Verwendung dieser Verbindungen als Farbstoff-Vorstufe in Färbemitteln für Keratinfasern

Aus der US-PS 2 273 564 ist ein Verfahren zur Herstellung von in 2-Stellung substituierten 1,4-Diaminobenzolen bekannt, bei dem das entsprechende Nitroanilin katalytisch hydriert wird. Die Herstellung des 1,4-Diamino-2-methoxymethyl-benzols nach diesem Verfahren ist jedoch recht aufwendig, da eine aufwendige Reinigung des Endproduktes erforderlich ist, die durch die Oxidationsempfindlichkeit der erhaltenen freien Base zusätzlich erschwert wird.
Aus der WO 99/11228 sind Färbemittel bekannt, welche eine Kombination aus 2-Chlor-6-methyl-3-aminophenol, einer Entwicklersubstanz (z.B. 2-(β-Hydroxyethyl)-p-phenylendiamin oder 2-Hydroxymethyl-p-phenylendiamin) und einer weiteren Kupplersubstanz aus der Gruppe bestehend aus m-Aminophenolen und m-Phenylendiaminen enthalten.

Es bestand daher die Aufgabe, ein einfacheres Herstellungsverfahren für das1,4-Diamino-2-methoxymethyl-benzol zur Verfügung zu stellen, das zusätzlich eine einfache Herstellung der oxidationsunempfindlicheren Salze ermöglicht.

Überraschenderweise wurde nunmehr gefunden, dass das 1,4-Diamino-2-methoxymethyl-benzol sowie dessen Salze der Formel (I) aus einem Nitrophenol der Formel (IV) durch Umsetzung in das entsprechende Phenoxyacetamid der Formel (V), anschließende Umlagerung zum Nitroanilin der Formel (VI) und Reduktion auf einfache Weise und in guten Ausbeuten erhältlich ist.

Gegenstand der vorliegenden Erfindung ist daher ein Verfahren zur Herstellung von 1,4-Diamino-2-methoxymethyl-benzol sowie dessen physiologisch verträglichen Salzen der Formel (I) 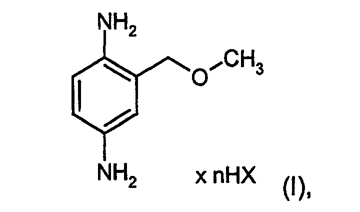 worin n einem Wert von 0 bis 2 entspricht und HX für eine anorganische oder organische Säure steht, in dem zunächst ein 2-Methoxymethyl-4-nitrophenol der Formel (IV) mit Chloracetamid zum 2-(2-Methoxymethyl-4-nitro-phenoxy)-acetamid der Formel (V) umgesetzt wird, sodann das 2-(2-Methoxymethyl-4-nitro-phenoxy)-acetamid der Formel (V) zum 2-Methoxymethyl-4-nitroanilin der Formel (VI) umgelagert wird und anschließend das 2-Methoxymethyl-4-nitroanilin der Formel (VI) katalytisch zum 1,4-Diamino-2-methoxymethyl-benzol der Formel (I) hydriert und gegebenenfalls (wenn n≠0) mit einer Säure zum entsprechenden Säureaddukt umgesetzt wird.

Als Säuren können anorganische oder organische Säuren eingesetzt werden, wobei die folgenden Säuren bevorzugt sind: Salzsäure, Schwefelsäure, Borsäure, Zitronensäure und Weinsäure. Besonders bevorzugt sind Salzsäure und Schwefelsäure.

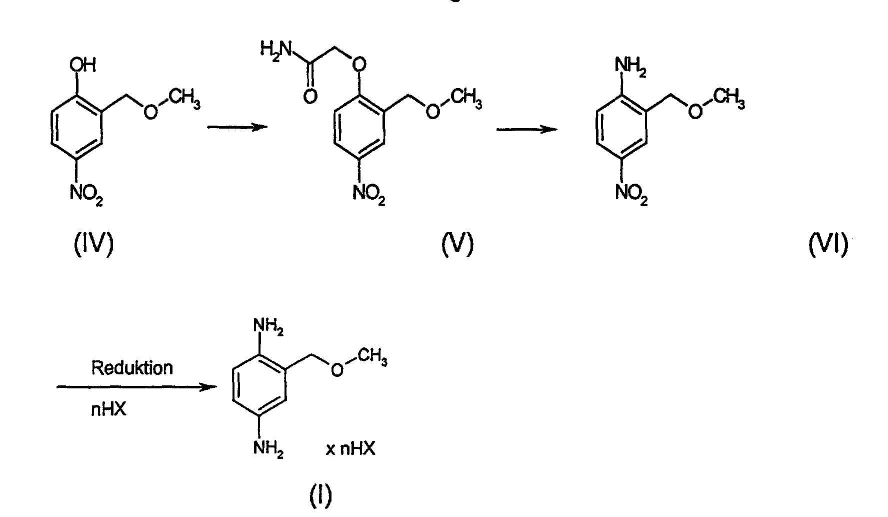

Das 2-Methoxymethyl-4-nitrophenol der Formel (IV) wird unter Erwärmen mit einem Halogenoacetamid umgesetzt. Insbesondere eignet sich hierfür Jodacetamid aber auch das etwas weniger reaktive Bromacetamid. Möchte man aus Kostengründen das billigere Chloracetamid verwenden, so ist es vorteilhaft, die Reaktion mit einem Jodid, beispielsweise Natriumjodid oder Kaliumjodid, zu katalysieren. Die zugesetzten Mengen an Jodid können im Hinblick auf eine hohe Umsatzrate weit über 10% (molar) betragen, wobei 50% (molar) sich als durchaus angemessen erwiesen haben. Die Reaktion kann in üblicher Weise in einem einfachen dipolaren aprotischen Lösungsmittel unter Rückflußtemperatur durchgeführt werden. Als dipolares aprotisches Lösungsmittel können zum Beispiel Aceton oder ein Di(C1-C6)alkylether von Mono- oder Polyethylenglykolen (beispielsweise Ethylenglykoldialkylether, Diethylenglykoldialkylether, Triethylenglykoldialkylether oder Polyethylenglykoldialkylether) verwendet werden, wobei die Verwendung von Aceton oder Di(C1-C6)-alkylethern von Mono- oder Polyethylenglykolen mit einer Siedetemperatur von unter 200 °C, und insbesondere die Verwendung von Aceton oder Ethylenglykoldimethylether, bevorzugt ist. Die Umlagerung des so erhaltenen 2-(2-Methoxymethyl-4-nitro-phenoxy)-acetamids der Formel (V) zum Nitroanilin (VI) erfolgt in Analogie zu W. R. Baker, J. Org. Chem. **1983,** *48*, 5140 durch Erwärmen der Verbindung der Formel (V) in Lösungsmitteln wie Dimethylformamid, Dimethylacetamid oder N-Methyl-pyrrolidon unter Zusatz einer Base. Als Basen eignen sich neben den in der zuvor genannten Literatur genannten Verbindungen auch Carbonate wie Natriumcarbonat oder Kaliumcarbonat. Da die Carbonate unter den Reaktionsbedingungen in Suspension vorliegen, ist es vorteilhaft, sie möglichst feinpulverig und im 2 bis 5fachen Überschuß einzusetzen. Die Reaktion kann in einem breiten Temperaturbereich durchgeführt werden, wobei unterhalb von 80°C der Umsatz niedrig ist, während oberhalb von 140°C die Reaktionsmischung durch Abbauprodukte dunkel gefärbt wird, so dass Temperaturen im Bereich von 80 bis 120°C bevorzugt sind. Die anschliessende katalytische Hydrierung zum 1,4-Diamino-2-methoxymethyl-benzol der Formel (I) erfolgt in üblicher Weise unter leicht erhöhtem Wasserstoffdruck bei Raumtemperatur oder leicht erhöhter Temperatur bei Verwendung eines Palladium/Aktivkohle-Katalysators. Ein besonderer Vorteil der erfindungsgemäßen Methode ist es, dass im letzten Reaktionsschritt keinerlei störende Nebenprodukte auftreten und so zusätzliche Reinigungsschritte nicht erforderlich sind. Das Produkt der Formel (I) wird wegen der hohen Oxidationsempfindlichkeit der freien Base vorzugsweise in Form seiner Salze mit einer organischen oder anorganischen Säure, beispielsweise Salzsäure oder Schwefelsäure, isoliert (n≠0). Die Säure gibt man unter Berücksichtigung der Anzahl der jeweiligen Säuregruppen im geringen Überschuß zu, um eine Protonierung beider Aminogruppen in Formel (I) zu erzielen. Bei einbasigen Säuren hat sich die Zugabe von 2 bis 2,5 Äquivalenten bewährt, bei zweibasigen Säuren ist eine Menge von 1 bis 1,5 Äquivalenten sinnvoll. Als Lösungsmittel sind zur Salzbildung Alkohole sowie Ether bevorzugt, da im allgemeinen die Säuren darin gut löslich sind und die Addukte daraus leicht kristallisierbar sind.

Die Verbindungen der Formel (I) eignen sich hervorragend als Farbstoff-Vorstufen im oxidativen System zum Färben von Keratinfasern.
Ein weiterer Gegenstand der gegenwärtigen Erfindung ist daher die Verwendung der Verbindung der Formel (I) in Färbemitteln für Keratinfasern, beispielsweise Wolle, Seide oder Haaren, insbesondere menschliche Haaren. Obwohl sich die Verbindungen der Formel (I) insbesondere für die Verwendung zur Färbung von Keratinfasern eignen, ist es prinzipiell auch möglich mit diesen Verbindungen andere natürliche oder synthetische Fasern, beispielsweise Baumwolle oder Nylon 66, zu färben.

Die Verbindung der Formel (I) kann sowohl alleine als auch in Kombination mit bestimmten bekannten Entwicklersubstanzen und/oder Kupplersubstanzen, die in oxidativen Färbesystemen zur Färbung von Fasermaterialien üblicherweise verwendet werden, eingesetzt werden. Als geeignete Kupplersubstanzen können insbesondere genannt werden: N-(3-Dimethylamino-phenyl)-hamstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1-(3-hydroxypropoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion, oder deren Salze.

Zur Herstellung insbesondere von Naturtönen und modischen Rottönen ist es besonders vorteilhaft, Verbindungen der Formel (I) in Kombination mit zusätzlichen Entwicklersubstanzen einzusetzen. Als Entwicklersubstanzen kommen Paraphenylendiamine, Paraaminophenole sowie 4,5-Diamino-pyrazole oder deren Salze in Betracht. Insbesondere sind die folgenden Entwicklersubstanzen zu nennen:
1,4-Diamino-benzol (p-Phenylendiamin), 1,4-Diamino-2-methyl-benzol (p-Toluylen-diamin), 1,4-Diamino-2,6-dimethyl-benzol, 1,4-Diamino-2,5-dimethyl-benzol, 1,4-Diamino-2,3-dimethyl-benzol, 2-Chlor-1,4-diaminobenzol, 4-Phenylamino-anilin, 4-Dimethylamino-anilin, 4-Diethylamino-anilin, 4-[Di(2-hydroxyethyl)amino]-anilin, 4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)-(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylamino-phenol, 4-Amino-2-(aminomethyl)-phenol, 4-Amino-2-[(2-hydroxyethyl)amino]methyl-phenol, 4-Amino-2-(methoxy-methyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methyl-phenyl)methyl]-1H-pyrazol, 1-[(4-Chlorphenyl)methyl]-4,5-diamino-1H-pyrazol, 4,5-Diamino-1-methyl-1H-pyrazol, 4,5-Diamino-3-methyl-1-phenyl-1H-pyrazol, 4,5-Diamino-1-(2-hydroxyethyl)-3-methyl-1H-pyrazol, 2-Amino-phenol, 2-Amino-6-methyl-phenol und 2-Amino-5-methyl-phenol, oder deren Salze.

Die Verbindungen der Formel (I) können selbstverständlich auch in Kombination mit üblichen direktziehenden anionischen, kationischen oder neutralen Farbstoffe verwendet werden. Zu den bevorzugten anionischen Farbstoffen zählen beispielsweise 6-Hydroxy-5-[(4-sulfophenyl)azo]-2-naphthalinsulfonsäure-dinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Mono- und Disulfonsäure) (C147005;D&C Yellow No. 10; Food Yellow No. 13, Acid Yellow No. 3), 5-Hydroxy-1-(4-sulfophenyl)-4-[(4-sulfophenyl)azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (C145350; Acid Yellow No. 73; D&C Yellow No. 8), 5-[(2,4-Dinitrophenyl)amino]-2-phenylamino-benzolsulfonsäure-natriumsalz (CI10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]phenyl)azo]-benzolsulfonsäure-natriumsalz (Cl20170; Acid Orange No. 24), 4-Hydroxy-3-[(4-sulfonaphth-1-yl)azo]-1-naphthalin-sulfonsäure-dinatriumsalz (CI14720; Acid Red No. 14), 6-Hydroxy-5-[(4-sulfonaphth-1-yl)azo]-2,4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäure-dinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (C145430;Acid Red No. 51), N-[6-(Diethylamino)-9-(2,4-disulfophenyl)-3H-xanthen-3-yliden]-N-ethylethanaminium-hydroxid, inneres Salz, Natriumsalz (CI45100; Acid Red No. 52), 8-[(4-(Phenylazo)phenyl)azo]-7-naphthol-1,3-disulfonsäure-dinatriumsalz (CI27290; Acid Red No. 73), 2',4',5',7'-Tetrabrom-3',6'-dihydroxyspiro[isobenzofuran-1(3H),9'-[9H]xanthen]-3-on-dinatriumsalz (C145380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro-[isobenzofuran-1(3H),9'(9H)-xanthen)-3-on-dinatriumsalz (CI45425; Acid Red No. 95), (2-Sulfophenyl)di[4-(ethyl((4-sulfophenyl)methyl)amino)phenyl]-carbenium-dinatriumsalz, betain (C142090; Acid Blue No. 9; FD&C Blue No. 1), 1,4-Bis[(2-sulfo-4-methylphenyl)amino]-9,10-anthrachinon-dinatriumsalz (Cl 61570; Acid Green No. 25), Bis[4-(dimethylamino)phenyl]-(3,7-disulfo-2-hydroxynaphth-1-yl)carbenium-inneres Salz, mononatriumsalz (C144090; Food Green No. 4; Acid Green No. 50), Bis[4-(diethylamino)phenyl](2,4-disulfophenyl)carbenium-inneres salz, Natriumsalz (2:1) (C142045; Food Blue No. 3; Acid Blue No. 1), Bis[4-(diethylamino)phenyl](5-hydroxy-2,4-disulfophenyl)carbenium-inneres salz, Calciumsalz (2:1) (C142051; Acid Blue No. 3), 1-Amino-4-(cyclohexylamino)-9,10-anthrachinon-2-sulfonsäure-natriumsalz (CI62045; Acid Blue No. 62), 2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalin-disulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1), 3-Hydroxy-4-(3-methyl-5-oxo-1-phenyl-4,5-dihydro-1H-pyrazol-4-ylazo)-naphthalin-1-sulfonsäure natriumsalz Chrom-Komplex (Acid Red No. 195).

Zu den bevorzugten kationischen Farbstoffen zählen beispielsweise 9-(Dimethylamino)-benzo[a]phenoxazin-7-ium-chlorid (CI51175; Basic Blue No. 6), Di[4-(diethylamino)phenyl][4-(ethylamino)naphthyl]carbenium-chlorid (CI42595; Basic Blue No. 7), 3,7-Di(dimethylamino)phenothiazin-5-ium-chlorid (CI52015; Basic Blue No. 9), Di[4-(dimethylamino)phenyl][4-(phenylamino)naphthyl]carbenium-chlorid (CI44045; Basic Blue No. 26), 2-[(4-(Ethyl(2-hydroxyethyl)amino)phenyl)azo]-6-methoxy-3-methyl-benzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)phenyl][4-(methylamino)phenyl]carbenium-chlorid (CI42535; Basic Violet No. 1), Tris(4-amino-3-methylphenyl)-carbenium-chlorid (CI42520; Basic Violet No. 2), Tris[4-(dimethylamino)phenyl]carbenium-chlorid (CI42555; Basic Violet No. 3), 2-[3,6-(Diethylamino)dibenzopyranium-9-yl]benzoesäure-chlorid (CI45170; Basic Violet No. 10), Di(4-aminophenyl)(4-amino-3-methylphenyl)carbenium-chlorid (CI42510; Basic Violet No. 14), 1,3-Bis[(2,4-diamino-5-methylphenyl)azo]-3-methylbenzol (CI21010;Basic Brown No. 4), 1-[(4-Aminophenyl)azo]-7-(trimethylammonio)-2-naphthol-chlorid (CI12250; Basic Brown No. 16), 1-[(4-Amino-2-nitrophenyl)azo]-7-(trimethylammonio)-2-naphthol-chlorid (CI12251; Basic Brown No. 17), 1-[(4-Amino-3-nitrophenyl)azo]-7-(trimethylammonio)-2-naphthol-chlorid (CI12251; Basic Brown No. 17), 3,7-Diamino-2,8-dimethyl-5-phenyl-phenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)azo]-pyrazol-5-on-chlorid (CI12719; Basic Yellow No. 57), Bis[4-(diethylamino)phenyl]phenylcarbenium-hydrogensulfat(1:1) (CI42040; Basic Green No. 1).

Zum besseren Farbausgleich und zur Erzeugung von speziellen Nuancen besonders bewährt haben sich nichtionische Farbstoffe aus der Gruppe 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis-[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 1-Amino-4-[(2-hydroxyethyl)amino]-2-nitrobenzol (HC Red No. 7), 2-Amino-4,6-dinitro-phenol, 2-Ethylamino-4,6-dinitrophenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitro-benzoesäure, 2-[(4-Amino-2-nitrophenyl)amino]-benzoesäure, 2-Chlor-6-methylamino-4-nitrophenol 2-Chlor-6-[(2-hydroxyethyl)amino]-4-nitrophenol, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxy-propyl)amino]-4-[methyl-(2-hydroxyethyl)amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1,4-Di[(2,3-dihydroxypropyl)amino]-9,10-anthrachinon, 1-[(2-Hydroxyethyl)amino]-4-methylamino-9,10-anthrachinon (CI61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8), 1-[(3-Aminopropyl)amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin, 2-((4-(Acetylamino)phenyl)azo)-4-methylphenol (CI11855; Disperse Yellow No. 3).

Aus der Gruppe der direktziehenden Farbstoffe besonders zu erwähnen sind 2-Amino-4,6-dinitrophenol, 2-Ethylamino-4,6-dinitrophenol, 2-[(2-Hydroxyethyl)amino]-4,6-dinitrophenol und Farbstoffe der allgemeinen Formel (VII), 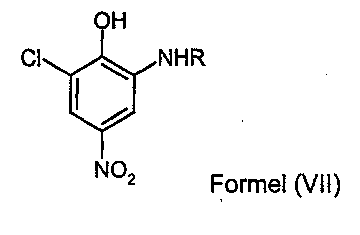 worin R Wasserstoff, Methyl, Ethyl oder Hydroxyethyl bedeutet.

Die vorstehend beschriebenen erfindungsgemäßen Kombinationen der Verbindungen der Formel (I) mit oxidativen Haarfarbvorstufen und/oder direktziehenden Farbstoffen werden zur Färbung in einer geeigneten Farbträgermasse appliziert.

Der Gegenstand der vorliegenden Erfindung betrifft daher auch ein Mittel zur oxidativen Färbung von Haaren, das durch Vermischen einer Farbträgermasse mit einem Oxidationsmittel unmittelbar vor der Anwendung hergestellt wird und dadurch gekennzeichnet ist, dass es mindestens eine Verbindung der Formel (I) sowie gegebenenfalls weitere Farbstoffvorstufen und/oder direktziehende Farbstoffe enthält.

In dem erfindungsgemäßen Mittel sind die Verbindungen der Formel (I) sowie die Farbvorstufen in einer Gesamtkonzentration von etwa 0,01 bis 10 Gewichtsprozent, vorzugsweise 0,2 und 6 Gewichtsprozent, enthalten. Die Gesamtkonzentration an direktziehenden Farbstoffen beträgt etwa 0,1 bis 10 Gewichtsprozent, vorzugsweise 0,1 bis 5 Gewichtsprozent.

Darüber hinaus können in der Farbträgermasse noch Antioxidantien, Parfümöle, Komplexbildner, Netzmittel, Emulgatoren, Penetrationsmittel, Puffersysteme, Konservierungsstoffe, Verdicker, Pflegestoffe und andere kosmetische Zusätze vorhanden sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze für Lösungen, Cremes, Emulsionen oder Gele sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionischen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet. Bezogen auf die Farbträgermasse sind dies zum Beispiel Netzmittel und Emulgatoren in Konzentrationen von etwa 0,5 bis 30 Gewichtsprozent, Verdicker in einer Menge von etwa 0,1 bis 25 Gewichtsprozent und die Pflegestoffe in einer Konzentration von etwa 0,1 bis 5,0 Gewichtsprozent.

Das gebrauchsfertige erfindungsgemäße Haarfärbemittel wird durch Mischen der Farbträgermasse mit einem flüssigen Oxidationsmittel unmittelbar vor der Anwendung hergestellt.

Als Oxidationsmittel kommen hauptsächlich Wasserstoffperoxid oder dessen Additionsverbindungen an Harnstoff, Melamin oder Natriumbromat in Form einer 1 bis 12prozentigen, vorzugsweise 6prozentigen, wässrigen Lösung in Betracht, wobei Wasserstoffperoxid besonders bevorzugt wird.

Die Farbträgermasse und das Oxidationsmittel werden hierbei im Gewichtsverhältnis von 5 zu 1 bis 1 zu 3 miteinander vermischt, wobei ein Gewichtsverhältnis von 1 bis 1 bis 1 zu 2 besonders bevorzugt ist.

Des pH-Wert des gebrauchsfertigen erfindungsgemäßen Haarfärbemittel stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis bestimmt wird. Der pH-Wert des gebrauchsfertigen Haarfärbemittels beträgt etwa 3 bis 11, bevorzugt zwischen 6 und 10,5 liegen.

Für die Einstellung des jeweiligen pH-Wertes der Farbträgermasse und des Oxidationsmittels können je nach dem gewünschten pH-Wert verdünnte organische oder anorganische Säuren, wie zum Beispiel Phosphorsäure, Ascorbinsäure und Milchsäure, oder Alkalien wie Monoethanolamin, Triethanolamin, 2-Amino-2-methyl-1-propanol, Ammoniak, Natronlauge, Kalilauge oder Tris(hydroxymethyl)aminomethan, verwendet werden.

Nach der Vermischung der vorstehend beschriebenen Farbträgermasse mit dem Oxidationsmittel wird eine für die Haarfärbebehandlung ausreichende Menge, je nach Haarfülle im allgemeinen etwa 60 bis 200 Gramm des erhaltenen gebrauchsfertigen Oxidationshaarfärbemittels auf das Haar aufgetragen.

Man läßt das erfindungsgemäße Haarfärbemittel etwa 10 bis 45 Minuten bei 15 bis 50 Grad Celsius, vorzugsweise 30 Minuten bei 40 Grad Celsius, auf das Haar einwirken und spült dann das Haar mit Wasser aus. Gegebenenfalls wird das Haar im Anschluß an diese Spülung mit einem Shampoo gewaschen und eventuell mit einer verdünnten schwachen organischen Säure, wie zum Beispiel Zitronensäure oder Weinsäure nachgespült. Abschließend wird das Haar getrocknet.

Die nachfolgenden Beispiele sollen den Gegenstand näher erläutern, ohne ihn jedoch auf diese Beispiele zu beschränken.

### Beispiele

### Beispiel 1: Herstellung von 1,4-Diamino-2-methoxymethyl-benzoldihydrochlorid

### Stufe 1: 2-(2-Methoxymethyl-4-nitro-phenoxy)-acetamid

25 g 2-Methoxymethyl-4-nitro-phenol, 14 g Chloracetamid, 18,9 g Kaliumcarbonat und 12,5 g Kaliumjodid werden in 150 ml Aceton 4 Stunden lang unter Rückfluß erhitzt. Anschließend läßt man die Reaktionsmischung auf Raumtemperatur abkühlen und gießt auf 450 ml Wasser. Der ausgefallene Niederschlag wird abfiltriert, mit Wasser gewaschen und anschließend getrocknet. Man erhält 20 g eines blaßgelben Produktes mit einem Schmelzpunkt von 157 bis 158°C.
¹H-NMR (DMSO-*d*_{*6*}): δ= 3,40 ppm (s, 3H); 4,60 ppm (s, 2H); 4,65 ppm (s, 2H); 7,09 ppm (d, ³J_{HH} = 9,1 Hz, 1H); 7,47 ppm (s breit, 2H); 8,15 ppm (d, ⁴J_{HH} = 2,55 Hz, 1H); 8,18 ppm (dd, ³J_{HH} = 9,1 Hz, ⁴J_{HH} = 2,55 Hz, 1H).

| CHN-Analyse: (C₁₀H₁₂N₂O₅; M = 240,21) | | | |
|---|---|---|---|
| | %C | %H | %N |
| ber. | 50,00 | 5,04 | 11,66 |
| gef. | 50,10 | 5,05 | 11,43 |

### Stufe 2: 2-Methoxymethyl-4-nitro-anilin

20 g des in Stufe 1 hergestellten 2-(2-Methoxymethyl-4-nitro-phenoxy)-acetamids werden in 55 ml N-Methyl-pyrrolidon gelöst, mit 29 g Kaliumcarbonat versetzt und 3 Stunden lang auf 100°C erhitzt. Anschließend läßt man auf Raumtemperatur abkühlen und entfernt das Lösungsmittel bei 60 bis 80°C im Vakuum. Der erhaltene Rückstand wird durch Zugabe von 100 ml Wasser zur Kristallisation gebracht. Man erhält 8,8 g eines gelblichen Prouktes mit einem Schmelzpunkt von 90 bis 92°C.
¹H-NMR (DMSO-*d*_{*6*}): δ= 3,30 ppm (s, 3H); 4,35 ppm (s, 2H); 6,51 ppm (s breit, 2H); 6,68 ppm (d, ³J_{HH} = 8,85 Hz, 1H); 7,92 ppm (dd, ³J_{HH} = 8,85 Hz, ⁴J_{HH} = 2,6 Hz, 1H); 7,98 ppm (d, ⁴J_{HH} = 2,6 Hz, 1H).

| CHN-Analyse: (C₈H₁₀N₂O₃; M = 182,18) | | | |
|---|---|---|---|
| | %C | %H | %N |
| ber. | 52,74 | 5,53 | 15,38 |
| gef. | 52,77 | 5,63 | 15,17 |

### Stufe 3: 1,4-Diamino-2-methoxymethyl-benzol-dihydrochlorid

8 g des in Stufe 2 hergestellten 2-Methoxymethyl-4-nitro-anilins werden in 100 ml Ethanol gelöst, mit 0,8 g Palladium (10% auf Aktivkohle) versetzt und unter leicht erhöhtem Wasserstoffdruck hydriert. Nach etwa 6 Stunden ist die Wasserstoffaufnahme beendet und man erhält nach der Entfernung des Katalysators durch Filtration eine farblose Lösung. Durch Einleiten von Chlorwasserstoffgas wird das Hydrochlorid ausgefällt. Man saugt den Niederschlag ab, wäscht mit wenig Ethanol nach und trocknet im Vakuum. Es werden 7,2 g hellbeige Kristalle mit einem Schmelzpunkt von >280°C erhalten.
¹H-NMR (DMSO-*d*_{*6*}): δ= 3,35 ppm (s, 3H); 4,55 ppm (s, 2H); 7,27 ppm (dd, ³J_{HH} = 8,30 Hz, ⁴J_{HH} = 2,30 Hz, 1H); 7,33 ppm (d, ⁴J_{HH} = 2,6 Hz, 1H); 7,36 ppm (d, ³J_{HH} = 8,30 Hz, 1H); 9,37 ppm (s, sehr breit, 6H + Wasser).

| CHN-Analyse: (C₈H₁₂N₂O x 2HCI; M = 225,12) | | | |
|---|---|---|---|
| | %C | %H | %N |
| ber. | 42,68 | 6,27 | 12,44 |
| gef. | 42,80 | 6,39 | 12,03 |

### Beispiel 2: Herstellung von 1,4-Diamino-2-methoxymethyl-benzolsulfat

3,65 g 2-Methoxymethyl-4-nitro-anilin aus Stufe 2 von Beispiel 1 werden in 35 ml Tetrahydrofuran gelöst, mit 0,3 g Palladium (10% auf Aktivkohle) versetzt und unter leicht erhöhtem Wasserstoffdruck hydriert. Nach etwa 5 Stunden ist die Wasserstoffaufnahme beendet und man erhält nach der Entfernung des Katalysators durch Filtration eine farblose Lösung. Durch Zugabe von 1,2 g Schwefelsäure in 50 ml Tetrahydrofuran wird das Säureaddukt ausgefällt. Man saugt das Salz ab, wäscht mit wenig Tetrahydrofuran nach und trocknet im Vakuum. Es werden 3,3 g hellbeige Kristalle mit einem Schmelzpunkt von 206 bis 212°C (unter Zersetzung) erhalten.
¹H-NMR (DMSO-*d*_{*6*}): δ= 3,30 ppm (s, 3H); 4,32 ppm (s, 2H); 6,79 ppm (d, ³J_{HH} = 8,55 Hz, 1H); 6,89 ppm (d verbreitert, ³J_{HH} = 8,55 Hz); 6,95 ppm (s verbreitert, 1H).

| CHNS-Analyse: (C8H12N20 x H2S04; M = 250,27) Unter Berücksichtigung von 1,21% Kristallwasser ergibt sich: | | | | |
|---|---|---|---|---|
| | %C | %H | %N | %S |
| ber. | 37,93 | 5,71 | 11,06 | 12,66 |
| gef. | 37,70 | 5,60 | 10,90 | 13,00 |

### Beispiel 3: Oxidationshaarfärbemittel, Creme basisch

| | |
|---|---|
| 15,00 g | Cetylstearylalkohol (50/50) |
| 5,00 g | Glycerinmonostearat |
| 2,00 g | Cocamide DEA |
| 10,00 g | Natriumlaurylethersulfat, 28%ige wässrige Lösung |
| 0,30 g | Ascorbinsäure |
| 0,40 g | Natriumsulfit |
| 4,50 g | Ammoniak, 25%ige wässrige Lösung |
| 0,56 g | 1,4-Diamino-2-methoxymethyl-benzol-dihydrochlorid |
| X g | Kupplersubstanz entsprechend Tabelle 1 |
| ad 100,00 g | Wasser, entmineralisiert |

Der pH-Wert der Cremes liegt zwischen 10 und 10,5.

Unmittelbar vor der Anwendung werden 100 Gramm der vorstehenden Farbträgermasse mit 100 Gramm einer 6%igen wäßrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40°C wird das Haar mit einem Shampoo gewaschen, mit Wasser gespült und getrocknet. Die erhaltenen Farbnuancen und Farbintensitäten sind in Tabelle 1 zusammengefaßt.

**Tabelle 1**

| **Beispiel** | **Kupplersubstanz** | | **Farbton** | **Intensität** |
|---|---|---|---|---|
| **3a** | 0,28 g | Resorcin | mittelblond | (++) |
| **3b** | 0,27 g | 3-Aminophenol | graubraun | (o) |
| **3c** | 0,31 g | 5-Amino-2-methylphenol | rotviolett | (++) |
| **3d** | 0,64 g | 5-((2-Hydroxyethyl)amino)-2-methoxyanilin-dihydrochlorid | blauschwarz | (++) |
| **3e** | 0,47 g | 6-Amino-3,4-dihydro-2H-1,4-benzoxazin-hydrochlorid | blauschwarz | (++) |
| **3f** | 0,59 g | 1,3-Diamino-4-methoxybenzol-sulfat | blau | (++) |
| **3g** | 0,36 g | 1-Chlor-2,4-dihydroxybenzol | gold | (++) |
| **3h** | 0,57 g | 3-Amino-6-methoxy-2-(methylamino)-pyridin-hydrochlorid | aubergine dunkel | (++) |
| **3i** | 0,35 g | 5-Hydroxy-1,3-benzodioxol | kastanie | (++) |
| **3j** | 0,43 g | 5-Amino-1,3-benzodioxol-hydrochlorid | dunkelbraun | (++) |
| **3k** | 0,44 g | 4-Methoxy-1-naphthol | aubergine | (o) |
| **3l** | 0,61 g | 3,5-Diamino-2,6-dimethoxy-pyridin-dihydrochlorid | blauschwarz | (++) |
| **3m** | 0,40 g | 1,7-Dihydroxynaphthalin | blauviolett | (o) |
| **3n** | 0,31 g | 1,3-Dihydroxy-2-methyl-benzol | rehbraun | (+) |
| **3o** | 0,45 g | 3-Dimethylamino-phenyl-harnstoff | anthrazit | (++) |
| **3p** | 0,33 g | 4-Hydroxyindol | schwarzviolett | (++) |
| **3q** | 0,39 g | 3-Amino-2-chlor-6-methylphenol | rotviolett | (++) |
| **3r** | 0,56 g | 2-Chlor-5-((2,2,2-trifluorethyl)amino)-phenol | blauviolett | (+) |
| **3s** | 0,45 g | 3-Amino-2,4-dichlorphenol | blauschwarz | (++) |
| **3t** | 0,36 g | 5-Amino-2-chlor-phenol | blauviolett | (++) |
| (o) = mittel, (+) = stark, (++) = sehr stark | | | | |

### Beispiel 4: Oxidationshaarfärbemittel, Creme sauer

| | |
|---|---|
| 15,00 g | Cetylstearylalkohol (50/50) |
| 5,00 g | Glycerinmonostearat |
| 2,00 g | Cocamide DEA |
| 10,00 g | Natriumlaurylethersulfat, 28%ige wässrige Lösung |
| 0,30 g | Ascorbinsäure |
| 0,40 g | Natriumsulfit |
| 0,62 g | 1,4-Diamino-2-methoxymethyl-benzol-sulfat |
| X g | Kupplersubstanz entsprechend Tabelle 2 |
| ad 100,00 g | Wasser, entmineralisiert |

Unmittelbar vor der Anwendung werden 100 Gramm der vorstehenden Farbträgermasse mit 100 Gramm einer 6%igen wäßrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40°C wird das Haar mit einem Farbpflegeshampoo gewaschen, mit Wasser gespült und getrocknet. Die erhaltenen Farbnuancen und Farbintensitäten sowie die pH-Werte der Färbemittel sind in Tabelle 2 zusammengefaßt.

**Tabelle 2**

| **Beispiel** | | **Kupplersubstanz** | **pH-Wert** | **Farbton** | **Intensität** |
|---|---|---|---|---|---|
| **4a** | 0,28 g | Resorcin | 6,6 | braun | (+) |
| **4b** | 0,27 g | 3-Aminophenol | 5,8 | mittel-blond | (o) |
| **4c** | 0,31 g | 5-Amino-2-methylphenol | 6,6 | rotviolett | (+) |
| **4d** | 0,64 g | 5-((2-Hydroxy-ethyl)amino)-2-methoxy-anilin-sulfat-hydrat | 6,7 | blau | (+) |
| (o) = mittel, (+) = stark, (++) = sehr stark | | | | | |

### Beispiel 5: Oxidationshaarfärbemittel in Gelform

| | |
|---|---|
| 15,00 g | Ölsäure |
| 3,00 g | Glycerin |
| 7,00 g | Isopropanol |
| 0,50 g | Ascorbinsäure |
| 0,40 g | Natriumsulfit |
| 0,40 g | Natriumhydroxid |
| 10,00 g | Ammoniak, 25%ige wässrige Lösung |
| 0,56 g | 1,4-Diamino-2-methoxymethyl-benzol-dihydrochlorid |
| X g | Kupplersubstanz entsprechend Tabelle 3 |
| ad 100,00 g | Wasser, entmineralisiert |

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40°C wird das Haar mit einem Shampoo gewaschen, mit Wasser gespült und getrocknet. Die erhaltenen Farbnuancen und Farbintensitäten sowie die pH-Werte der Färbemittel sind in Tabelle 3 zusammengefaßt.

**Tabelle 3**

| **Beispiel** | | **Kupplersubstanz** | **pH-Wert** | **Farbton** | **Intensität** |
|---|---|---|---|---|---|
| **5a** | 0,28 g | Resorcin | 10,0 | mittelblond | (++) |
| **5b** | 0,27 g | 3-Aminophenol | 9,9 | palisander | (+) |
| **5c** | 0,31 g | 5-Amino-2-methylphenol | 10,2 | rotviolett | (+) |
| **5d** | 0,64 g | 5-((2-Hydroxy-ethyl)amino)-2-methoxy-anilin-sulfat-hydrat | 10,6 | blau | (++) |
| (o) = mittel, (+) = stark, (++) = sehr stark | | | | | |

### Beispiel 6: Haarfärbelösung mit einem basischen pH-Wert

| | |
|---|---|
| 10,00 g | Ethanol |
| 10,00 g | Natriumlaurylethersulfat, 28%ige wässrige Lösung |
| 10,00 g | Ammoniak, 25%ige wässrige Lösung |
| 0,30 g | Ascorbinsäure |
| 0,62 g | 1,4-Diamino-2-methoxymethyl-benzol-sulfat |
| X g | Kupplersubstanz entsprechend Tabelle 4 |
| ad 100,00 g | Wasser, entmineralisiert |

Zur Anwendung werden 10 Gramm der vorstehenden Haarfärbelösung mit 10 Gramm einer 6%igen wäßrigen Wasserstoffperoxidlösung gemischt. Das so erhaltene gebrauchsfertige Oxidationshaarfärbemittel wird anschließend auf gebleichte Haare aufgetragen. Nach einer Einwirkzeit von 30 Minuten bei 40°C wird das Haar mit einem Farbpflegeshampoo gewaschen, mit Wasser gespült und getrocknet.
Die erhaltenen Farbnuancen und Farbintensitäten sowie die pH-Werte der Färbemittel sind in Tabelle 4 zusammengefaßt.

**Tabelle 4**

| **Beispiel** | | **Kupplersubstanz** | **pH-Wert** | **Farbton** | **Intensität** |
|---|---|---|---|---|---|
| **6a** | 0,6 6 g | 1,3-Diamino-4-(2-hydroxy-ethoxy)-benzol-sulfat | 10,7 | blau | (+) |
| **6b** | 0,39 g | 3-Amino-2-chlor-6-methyl-phenol | 10,5 | violett | (+) |
| **6c** | 0,45 g | 3-Amino-2,4-dichlor-phenol | 10,6 | aubergine | (+) |
| **6d** | 0,56 g | 2-Chlor-5-((2,2,2-trifluor-ethyl)-amino)-phenol | 10,7 | blau | (+) |
| **6e** | 0,61 g | 3-Amino-6-methoxy-2-(methyl-amino)-pyridin-dihydrochlorid | 10,6 | blaugrün | (+) |
| **6f** | 0,36 g | 1-Naphthol | 10,9 | blauviolett | (+) |
| (o) = mittel, (+) = stark, (++) = sehr stark | | | | | |

### Beispiel 7: Haarfärbelösung mit einem basischen pH-Wert

| | |
|---|---|
| 10,00 g | Ethanol |
| 10,00 g | Natriumlaurylethersulfat, 28%ige wässrige Lösung |
| 10,00 g | Ammoniak, 25%ige wässrige Lösung |
| 0,30 g | Ascorbinsäure |
| 0,38 g | 1,4-Diamino-2-methoxymethyl-benzol-dihydrochlorid |
| X g | Kupplersubstanz entsprechend Tabelle 5 |
| ad 100,00 g | Wasser, entmineralisiert |

Zur Anwendung werden 10 Gramm der vorstehenden Haarfärbelösung mit 10 Gramm einer 6%igen wäßrigen Wasserstoffperoxidlösung gemischt. Das so erhaltene gebrauchsfertige Oxidationshaarfärbemittel wird anschließend auf gebleichte Haare aufgetragen. Nach einer Einwirkzeit von 30 Minuten bei 40°C wird das Haar mit einem Farbpflegeshampoo gewaschen, mit Wasser gespült und getrocknet.
Die erhaltenen Farbnuancen und Farbintensitäten sowie die pH-Werte der Färbemittel sind in Tabelle 5 zusammengefaßt.

**Tabelle 5**

| **Beispiel** | | **Kupplersubstanz** | **pH-Wert** | **Farbton** | **Intensität** |
|---|---|---|---|---|---|
| **7a** | 0,40 g | 1,3-Diamino-4-(2-hydroxy-ethoxy)-benzol-dihydrochlorid | 10,5 | blau | (+) |
| **7b** | 0,26 g | 3-Amino-2-chlor-6-methyl-phenol | 10,5 | violett | (o) |
| **7c** | 0,30 g | 3-Amino-2,4-dichlor-phenol | 10,6 | aubergine | (o) |
| **7d** | 0,37g | 2-Chlor-5-((2,2,2-trifluorethyl)-amino)-phenol | 10,4 | blau | |
| **7e** | 0,40 g | 3-Amino-6-methoxy-2-(methylamino)-pyridin-dihydrochlorid | 10,4 | blaugrün | (o) |
| **7f** | 0,24 g | 1-Naphthol | 10,5 | blauviolett | (+) |
| (o) = mittel, (+) = stark, (++) = sehr stark | | | | | |

### Beispiel 8: Färbemittel

Eine gebrauchsfertige Creme nach Beispiel 3 wird auf die in Tabelle 6 angeführten Textilien aufgetragen, nach einer Einwirkungszeit von 30 Minuten bei 40°C wird neutralisiert und mit Wasser gründlich ausgewaschen.

**Tabelle 6**

| **Beispiel** | **Fasermaterial** | **Färbemasse gemäß Beispiel 3a** | **Färbemasse gemäß Beispiel 3b** | **Färbemasse gemäß Beispiel 3c** | **Färbemasse gemäß Beispiel 3d** |
|---|---|---|---|---|---|
| **8a** | Baumwolle | mittelbraun (+) | hellbraun (+) | rose (o) | blaugrau (+) |
| **8b** | Seide | mittelbraun (+) | braun (++) | rose (++) | blau (+) |
| **8c** | Wolle | caramel (++) | dunkelbraun (++) | rotviolett (++) | blauschwarz (++) |
| **8d** | Nylon 66 | mittelblond (+) | hellbraun (+) | rotbraun (+) | aubergine (+) |
| (o) = mittel, (+) = stark, (++) = sehr stark | | | | | |

### Beispiel 9: Haarfärbemittel in Gelform

| | |
|---|---|
| 15,25 g | Ölsäure |
| 3,15 g | Glycerin |
| 7,80 g | Ethanol |
| 0,50 g | Ascorbinsäure |
| X g | Farbstoff gemäß Tabelle 7 |
| 10,40 g | Ammoniak, 25%ige wässrige Lösung |
| ad 100,00 g | Wasser, entmineralisiert |

Zur Anwendung werden 100 Gramm der vorstehenden Farbträgermasse mit 100 Gramm einer 6%igen wäßrigen Wasserstoffperoxidlösung gemischt. Das so erhaltene gebrauchsfertige Oxidationshaarfärbemittel wird anschließend auf gebleichte Haare aufgetragen. Nach einer Einwirkzeit von 30 Minuten bei 40°C wird das Haar mit einem Farbpflegeshampoo gewaschen, mit Wasser gespült und sodann getrocknet.
Die erhaltenen Farbergebnisse sind in Tabelle 7 zusammengefaßt.

### Beispiel 10: Haarfärbemittel, Creme basisch

| | |
|---|---|
| 15,00 g | Cetylstearylalkohol (50/50) |
| 5,00 g | Glycerinmonostearat |
| 10,00 g | Natriumlaurylethersulfat, 28%ige wässrige Lösung |
| 0,30 g | Natriumsulfit |
| 5,00 g | Ammoniak, 25%ige wässrige Lösung |
| X g | Farbstoff gemäß Tabelle 8 |
| ad 100,00 g | Wasser, entmineralisiert |

Unmittelbar vor der Anwendung werden 100 Gramm der vorstehenden Farbträgermasse mit 100 Gramm einer 6%igen wäßrigen Wasserstoffperoxidlösung vermischt. Das so erhaltene gebrauchsfertige Oxidationshaarfärbemittel wird anschließend Menge auf mittelblonde Naturhaare aufgetragen. Nach einer Einwirkzeit von 30 Minuten bei 40°C wird das Haar mit einem Farbpflegeshampoo gewaschen, mit Wasser gespült und sodann getrocknet.
Die erhaltenen Farbergebnisse sind in Tabelle 8 zusammengefaßt.

**Tabelle 7**

| **Farbstoff / Beispiel** | **9a** | **9b** | **9c** | **9d** |
|---|---|---|---|---|
| 1,4-Diamino-2-methoxymethylbenzol-dihydrochlorid | 0,26 g | 1,00 g | 2,25 g | 1,30 g |
| 1,4-Diamino-2-methyl-benzolsulfat | - | 1,00 g | - | - |
| 1,4-Diamino-2-(2-hydroxyethyl)benzol-sulfat | 0,20 g - | | 0,42 g | - |
| 4-Amino-3-methyl-phenol | 0,05 g | 0,15 g | 0,15 g | - |
| 4,5-Diamino-1-(2-hydroxyethyl)-1 H-pyrazol-sulfat | 0,50 g | - | 0,10 g | - |
| 1,3-Diamino-4-(2-hydroxyethoxy)benzol-sulfat | - | 0,20 g | - | |
| 1,3-Diamino-4-(3-hydroxypropoxy)-benzoldihydrochlorid | - | - | 0,52 g | 0,10 g |
| 5-((2-Hydroxyethyl)amino)-2-methoxy-anilin-sulfat | - | 0,10 g | - | - |
| 1-(2-Hydroxyethylamino)-3,4-methylendioxobenzol | - | 0,20 g | 0,34 g | 0,50 g |
| Resorcin | 0,15 g | 0,21 g | 0,21 g | - |
| 2-Methylresorcin | - | 0,25 g | 0,10 g | - |
| 5-Amino-2-methylphenol | - | 0,20 g | 0,10 g | 0,18 g |
| 3-Amino-phenol | 0,34 g | 0,52 g | 0,52 g | - |
| 2-Amino-6-chlor-4-nitro-phenol | - | 0,24 g | 0,24 g | 0,16 g |
| 2-Chlor-6-(ethylamino)-4-nitrophenol | 0,10 g | - | 0,78 g | - |
| **erhaltene Färbung** | rubinrot | dunkelbraun | palisander | mittelbraun |

**Tabelle 8**

| **Farbstoff / Beispiel** | **10a** | **10b** | **10c** | **10d** |
|---|---|---|---|---|
| 1,4-Diamino-2-methoxymethyl-benzol-sulfat | 3,00 g | 0,13 g | 0,13 g | 1,45 g |
| 5-((2-Hydroxyethyl)amino)-2-methoxy-anilin-sulfat | 0,36 g | - | - | - |
| 1,4-Diamino-2-(2-hydroxyethyl)-benzol-sulfat | 0,13 g | - | 0,64 g | - |
| 1-(2-Hydroxyethylamino)-3,4-methylendioxobenzol | 0,20 g | 0,10 g | 0,10 g | 0,43 g |
| 4-Amino-3-methyl-phenol | 0,17 g | 0,83 g | 0,83 g | 0,07 g |
| 2-Methylresorcin | 0,30 g | 0,30 g | 0,30 g | - |
| 3-Amino-phenol | 0,56 g | - | - | 0,16 g |
| 2-Amino-6-chlor-4-nitrophenol | 0,35 g | 0,27 g | 0,27 g | 0,10 g |
| 1-Naphthol | - | 0,04 g | 0,04 g | - |
| **erhaltene Färbung** | schwarz | mittelbraun | dunkelbraun | dunkelbraun |

## Patentansprüche

1. Verfahren zur Herstellung von 1,4-Diamino-2-methoxymethylbenzol und dessen physiologisch verträglichen Salzen der Formel (I) 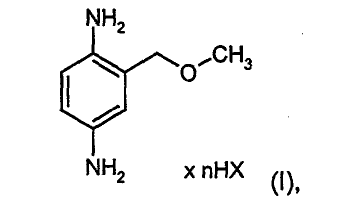 worin n einem Wert von 0 bis 2 entspricht und HX für eine anorganische oder organische Säure steht, in dem zunächst ein 2-Methoxymethyl-4-nitrophenol der Formel (IV) mit einem Halogenacetamid zum 2-(2-Methoxymethyl-4-nitro-phenoxy)-acetamid umgesetzt wird, sodann das 2-(2-Methoxymethyl-4-nitro-phenoxy)-acetamid zum 2-Methoxymethyl- 4-nitroanilin umgelagert wird und anschließend das 2-Methoxymethyl-4-nitroanilin katalytisch zum 1,4-Diamino-2-methoxymethyl-benzol der Formel (I) hydriert wird und wenn gilt n≠0 anschließend mit einer anorganischen oder organischen Säure umgesetzt wird.

2. Verfahren nach Anspruch 1, **dadurch gekennzeichnet, dass** das Halogenacetamid ausgweählt ist aus Bromacetamid und Jodacetamid.

3. Verfahren nach Anspruch 1 oder 2, **dadurch gekennzeichnet, dass** die Säure ausgewählt ist aus Salzsäure und Schwefelsäure.

4. Verwendung der Verbindung gemäß Formel (I) in Färbemitteln für Keratinfasern.

5. Mittel zur oxidativen Färbung von Haaren, welches durch Vermischen einer Farbträgermasse mit einem Oxidationsmittel unmittelbar vor der Anwendung hergestellt wird und **dadurch gekennzeichnet ist, dass** es mindestens eine Verbindung der Formel (I) sowie gegebenenfalls weitere Farbstoffvorstufen und/oder direktziehende Farbstoffe enthält.

6. Mittel nach Anspruch 5, **dadurch gekennzeichnet, dass** es die Verbindungen der Formel (I) sowie die weiteren Farbstoffvorstufen in einer Gesamtmenge von 0,01 bis 10 Gewichtsprozent enthält.

7. Mittel nach Anspruch 5 oder 6, **dadurch gekennzeichnet, dass** die direktziehenden Farbstoffe in einer Gesamtmenge von 0,1 bis 10 Gewichtsprozent enthalten sind.

8. Mittel nach einem der Ansprüche 5 bis 7, **dadurch gekennzeichnet, dass** es unmittelbar vor der Anwendung durch Vermischen einer Farbträgermasse mit einem Oxidationsmittel in einem Gewichtsverhältnis von 5:1 bis 1:3 hergestellt wird.

## Claims

1. Process for the preparation of 1,4-diamino-2-methoxymethylbenzene and physiologically compatible salts thereof of the formula (I) 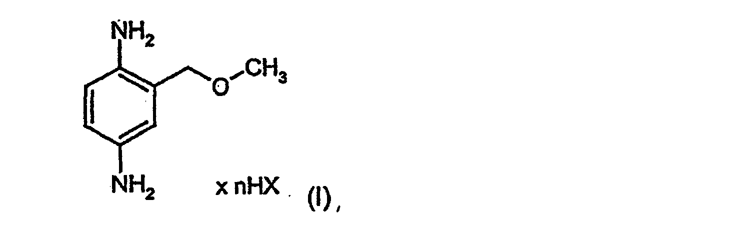 in which n corresponds to a value from 0 to 2, and HX is an inorganic or organic acid in which firstly a 2-methoxymethyl-4-nitrophenol of the formula (IV) is reacted with a haloacetamide to give 2-(2-methoxymethyl-4-nitrophenoxy)acetamide, then the 2-(2-methoxymethyl-4-nitrophenoxy)acetamide is rearranged to give 2-methoxymethyl-4-nitroaniline and then the 2-methoxymethyl-4-nitroaniline is hydrogenated catalytically to give 1,4-diamino-2-methoxymethylbenzene of the formula (I), and if n ≠ 0, is then reacted with an inorganic or organic acid.

2. Process according to Claim 1, **characterized in that** the haloacetamide is selected from bromoacetamide and iodoacetamide.

3. Process according to 1 Claim 1 or 2, **characterized in that** the acid is chosen from hydrochloric acid and sulphuric acid.

4. Use of the compound according to formula (I) in colorants for keratin fibres.

5. Agent for the oxidative colouring of hair, which is prepared by mixing a colour carrier mass with an oxidizing agent directly prior to use and **characterized in that** it comprises at least one compound of the formula (I), and optionally further dye precursors and/or direct dyes.

6. Agent according to Claim 5, **characterized in that** it comprises the compounds of the formula (I), and the further dye precursors in a total amount of from 0.01 to 10% by weight.

7. Agent according to Claim 5 or 6, **characterized in that** the direct dyes are present in a total amount of from 0.1 to 10% by weight.

8. Agent according to one of Claims 5 to 7, **characterized in that** it is prepared directly prior to use by mixing a colour carrier mass with an oxidizing agent in a weight ratio of from 5:1 to 1:3.

## Revendications

1. Procédé pour la préparation de 1,4-diamino-2-méthoxyméthylbenzène et ses sels physiologiquement acceptables de formule (1) 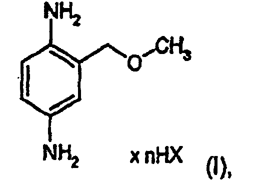 dans laquelle n vaut une valeur de 0 à 2 et HX représente un acide inorganique ou organique, en ce qu'on fait d'abord réagir un 2-méthoxyméthyl-4-nitrophénol de formule (IV) avec un halogénoacétamide en 2-(2-méthoxyméthyl-4-nitrophénoxy)acétamide puis le 2-(2-méthoxyméthyl-4-nitrophénoxy)acétamide est réarrangé en 2-méthoxyméthyl-4-nitroaniline, puis on hydrogène la 2-méthoxyméthyl-4-nitroaniline catalytiquement en 1,4-diamino-2-méthoxyméthylbenzène de formule (1) et, lorsque n ≠ 0, on transforme ensuite avec un acide inorganique ou organique.

2. Procédé selon la revendication 1, **caractérisé en ce que** l'halogénoacétamide est choisi parmi le bromoacétamide et l'iodoacétamide.

3. Procédé selon la revendication 1 ou 2, **caractérisé en ce que** l'acide est choisi parmi l'acide chlorhydrique et l'acide sulfurique.

4. Utilisation du composé selon la formule (1) dans des teintures pour fibres kératiniques.

5. Agent pour la teinture par oxydation de cheveux, qui est préparé par mélange d'une masse support de teinture avec un oxydant juste avant l'utilisation et qui est **caractérisé en ce qu'**il contient au moins un composé de formule (1) ainsi que le cas échéant d'autres précurseurs de colorants et/ou des colorants directs.

6. Agent selon la revendication 5, **caractérisé en ce qu'**il contient les composés de formule (1) ainsi que les autres précurseurs de colorants en une quantité totale de 0,01 à 10% en poids.

7. Agent selon la revendication 5 ou 6, **caractérisé en ce que** les colorants directs sont contenus en une quantité totale de 0,1 à 10% en poids.

8. Agent selon l'une quelconque des revendications 5 à 7, **caractérisé en ce qu'**il est préparé juste avant l'utilisation par mélange d'une masse support de teinture avec un oxydant dans un rapport pondéral de 5:1 à 1:3.
